# EUROPEAN PATENT APPLICATION

(11) **EP 4 659 715 A1**
(43) Date of publication of application: **10.12.2025**
(21) Application number: 24750380.8
(22) Date of filing: 01.02.2024
(51) Int. Cl.: A61F 2/844, A61F 2/95, A61M 29/00

(54) **PRODUCTION METHOD FOR STRING-EQUIPPED STENT, STRING-EQUIPPED STENT, AND STENT DELIVERY DEVICE**

(30) Priority: 03.02.2023 JP 2023015509; 22.01.2024 JP 2024007199
(71) Applicant: SB-Kawasumi Laboratories, Inc., Kawasaki-shi, Kanagawa 210-8602 (JP); Sumitomo Bakelite Co., Ltd., Shinagawa-ku Tokyo 140-0002 (JP)
(72) Inventor: MUKAI, Tomokazu, Kawasaki-shi, Kanagawa 210-8602 (JP); MIYAHISA, Masako, Kawasaki-shi, Kanagawa 210-8602 (JP)
(74) Representative: Müller-Boré & Partner Patentanwälte PartG mbB
(86) International application number: PCT/JP2024/003227
(87) International publication number: WO 2024/162431

(57) **Abstract**

A manufacturing method of a stent with a string 4 includes a first knot forming step of forming a first bent portion 12a at a string 6 and forming a first knot 11a by passing a part on the other end side 6h of the string 6 with respect to the first bent portion 12a between the first bent portion 12a and a stent 5 in one circumferential direction around the stent 5 and a second knot forming step of forming a second bent portion 12b at the part passed between the first bent portion 12a and the stent 5 and forming a second knot 11b by passing a part on the other end side 6h of the string 6 with respect to the second bent portion 12b between the second bent portion 12b and the stent 5 in the other circumferential direction around the stent 5, which is an opposite direction to a direction of passing the first bent portion 12a. The first knot 11a is formed on a first side 5x when viewed in an axial direction AX of the stent 5, and the second knot 11b is formed on a second side 5y, which is an opposite side to the first side 5x.

## Description

### Technical Field

The present invention relates to a stent with a string that is indwelled inside a biological lumen, and relates to a manufacturing method of a stent with a string, a stent with a string, and a stent delivery device.

### Background Art

A stent is used by being indwelled in a narrowing portion, an obstruction portion, an adhesion portion (hereinafter, referred to as a "narrowing portion or the like" in some cases) or the like, which is generated in a biological lumen. The stent is generally formed as a small metal tube having a mesh shape that can be expanded. The stent includes a self-expanding stent made of a superelastic metal. This type of self-expanding stent is transported to the adhesion portion or the like in a state of being reduced in diameter by being tied with a string from an outer periphery and then expands by releasing the restraint by the string.

For example, Patent Document 1 describes a stent held in a diameter-reduced state by a string (referred to as a wire in the same document) and discloses a tying method of a string in which the stent can be easily expanded by pulling a wire on a moving end side of the string. The string that ties the stent in Patent Document 1 has a part that extends in an axial direction of the stent, a part that extends in a circumferential direction of the stent, and a cross-shaped intersection portion that intersects the parts by tying and knotting the parts. In Document 1, a plurality of intersection portions are linearly arranged in the axial direction at a single in the circumferential direction of the stent.

### Prior Art Document

### Patent Document

[Patent Document 1] JP-A-2005-304792

### Summary of Invention

### Technical Problem

When the stent expands, a reaction force from a narrowing portion or the like in contact with the stent is applied to the stent. In the string of Patent Document 1, as described above, the plurality of intersection portions are linearly arranged in the axial direction at a single place in the circumferential direction of the stent.

For this reason, according to the tying method of a string to the stent in Patent Document 1, when the stent expands, a single place in the circumferential direction of the stent expands first. For this reason, a part of the stent that has expanded first presses the narrowing portion or the like first, and a reaction force is applied to the part from the narrowing portion or the like.

Therefore, in the technique of Patent Document 1, there is room for improvement in accurately placing the stent at a target position for indwelling without being affected by the reaction force from the narrowing portion or the like.

The present invention has been devised in view of the problems described above and provides a manufacturing method of a stent with a string, a stent with a string, and a stent delivery device, which can accurately indwell a stent at a target position for indwelling.

### Solution to Problem

According to an aspect of the present invention, there is provided a manufacturing method of a stent with a string in which a stent that is passed in a tubular portion in a body of a subject and that is expanded by elasticity of the stent to be able to indwell within the tubular portion and a string that ties a periphery of the stent such that expansion of the contracted stent is limited to maintain a contracted state when inserting the stent into the tubular portion are prepared, and a plurality of knots from one end side of the string to the other end side of the string are formed, the manufacturing method including a first knot forming step of forming a first bent portion at the string and forming a first knot by aligning the first bent portion with the stent and by passing a part on the other end side of the string with respect to the first bent portion between the first bent portion and the stent in one circumferential direction around the stent and a second knot forming step of forming a second bent portion at the part passed between the first bent portion and the stent and forming a second knot by passing a part on the other end side of the string with respect to the second bent portion between the second bent portion and the stent in the other circumferential direction around the stent, which is an opposite direction to a direction of passing the first bent portion, in which in the first knot forming step, the first knot is formed on a first side when viewed in an axial direction of the stent, in the second knot forming step, the second knot is formed on a second side, which is an opposite side to the first side, when viewed in the axial direction of the stent, and the stent is tied with the string by alternately repeating the first knot forming step and the second knot forming step.

According to another aspect of the present invention, there is provided a stent with a string including a stent that is configured by a mesh body, that is passed in a tubular portion in a body of a subject, and that is expanded by elasticity of the stent to be able to indwell within the tubular portion and a string that ties a periphery of the stent such that expansion of the contracted stent is limited to maintain a contracted state when inserting the stent into the tubular portion, in which the string includes a first knot formed on a first side when viewed in an axial direction of the stent, and a second knot formed on a second side, which is an opposite side to the first side, when viewed in the axial direction of the stent.

According to still another aspect of the present invention, there is provided a stent delivery device including the stent with a string and a delivery portion that delivers the stent with a string.

### Advantageous Effects of Invention

With the manufacturing method of a stent with a string, the stent with a string, and the stent delivery device according to the present invention, the stent can be accurately indwelled at a target position for indwelling.

### Brief Description of Drawings

Fig. 1 is a schematic overall view showing a stent delivery device according to the present embodiment.
Fig. 2 is a schematic view showing a tying region formed by a string for a stent.
Fig. 3 is a schematic view showing a state where a temporary fixing portion having a first bent portion is formed.
Fig. 4 is a schematic view showing a state where a second bent portion is formed after the state shown in Fig. 3.
Fig. 5 is a schematic view showing a state where a first bent portion is formed after the state shown in Fig. 4.
Fig. 6 is a schematic view showing a state where the second bent portion is formed after the state shown in Fig. 5.
Fig. 7 is a schematic view showing a state where the first bent portion is formed after the state shown in Fig. 6.
Fig. 8 is a view showing a procedure of forming the temporary fixing portion and is a schematic view showing a state where the string having the first bent portion is aligned with the stent.
Fig. 9 is a schematic view showing Fig. 8 viewed in an axial direction.
Fig. 10 is a schematic view showing a state where a third bent portion is formed after the state shown in Fig. 8.
Fig. 11 is a schematic view of Fig. 10 viewed in the axial direction.
Fig. 12 is a schematic view showing a state where the first bent portion is formed after the state shown in Fig. 10.
Fig. 13 is a schematic view of Fig. 12 viewed in the axial direction.
Fig. 14 is a view showing a procedure of forming a temporary fixing portion according to a modification example and is a schematic view showing a state where a fourth bent portion is formed after the state shown in Fig. 8.
Fig. 15 is a schematic view of Fig. 14 viewed in the axial direction.

### Description of Embodiments

Hereinafter, an embodiment of the present invention will be described with reference to the drawings. The embodiment to be described below is merely an example for facilitating understanding of the present invention and does not limit the present invention. That is, the shape, dimensions, disposition, and the like of members to be described below can be changed and improved without departing from the spirit of the present invention, and it is obvious that the present invention includes equivalents thereof.

It is not necessary for various types of components of the present invention to be individually independent, and it is allowed that a plurality of components are configured as a single component, one component is formed by being divided into a plurality of components, a certain component is a part of another component, a part of a certain component and a part of another component overlap each other, and the like.

In addition, in all the drawings, the same components are assigned with the same reference signs, and redundant description will be omitted as appropriate.

The thickness and size of each part shown in the drawings of the present application are changed as appropriate for ease of description and are not limited to those matching the actual scale.

In addition, a side close to an operator using the delivery device 1 will be referred to as a proximal side, and a side far from the operator will be referred to as a distal side.

A stent 5 to be described later is formed by a plurality of wires intersecting in a mesh shape, but for simplification of illustration, mesh-shaped portions are omitted, and only the outer shape as a whole is shown.

### <Overview>

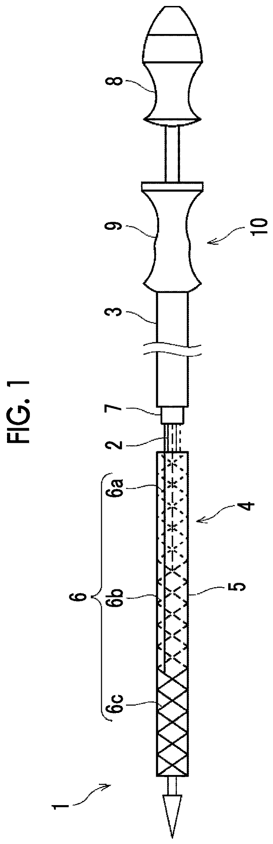

First, an overview of a manufacturing method of a stent with a string 4 according to the present embodiment will be mainly described with reference to Figs. 1 to 7.

Fig. 1 is a schematic overall view showing a stent delivery device (delivery device 1) according to the present embodiment.

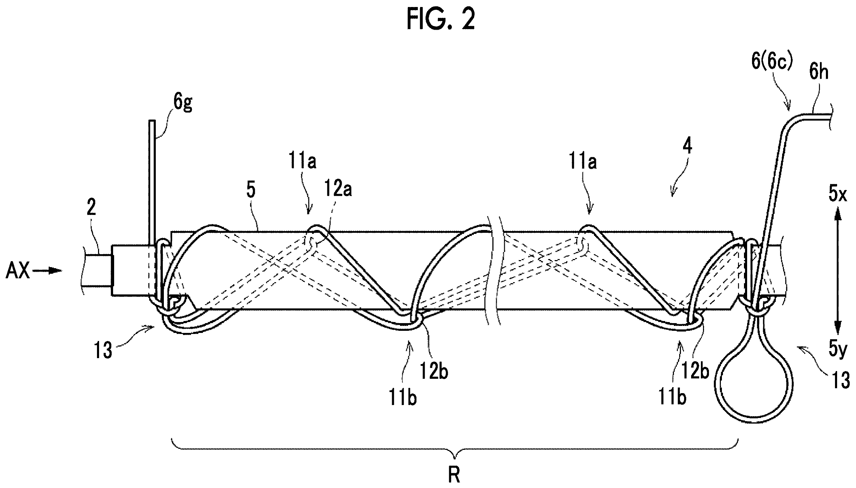

Fig. 2 is a schematic view showing a tying region R formed by a string 6 for the stent 5.

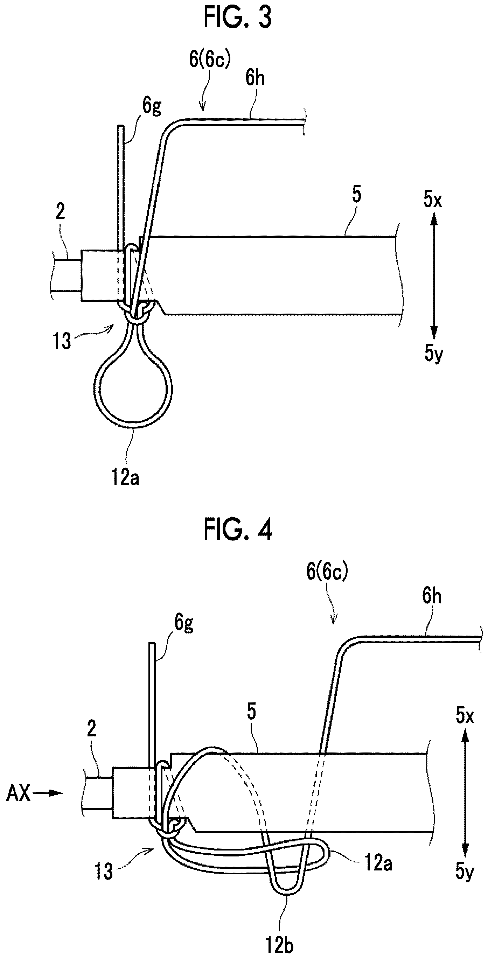

Fig. 3 is a schematic view showing a state where a temporary fixing portion 13 having a first bent portion 12a is formed. Fig. 4 is a schematic view showing a state where a second bent portion 12b is formed after the state shown in Fig. 3.

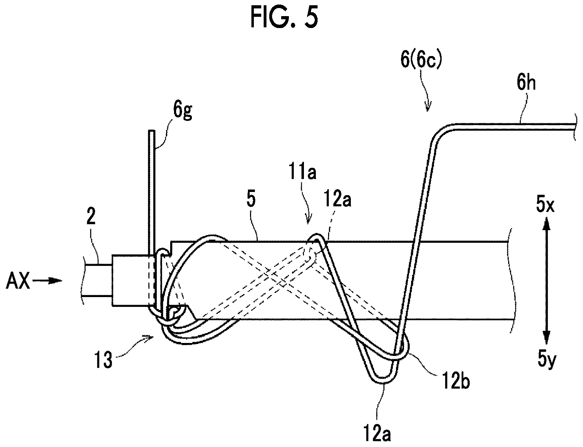

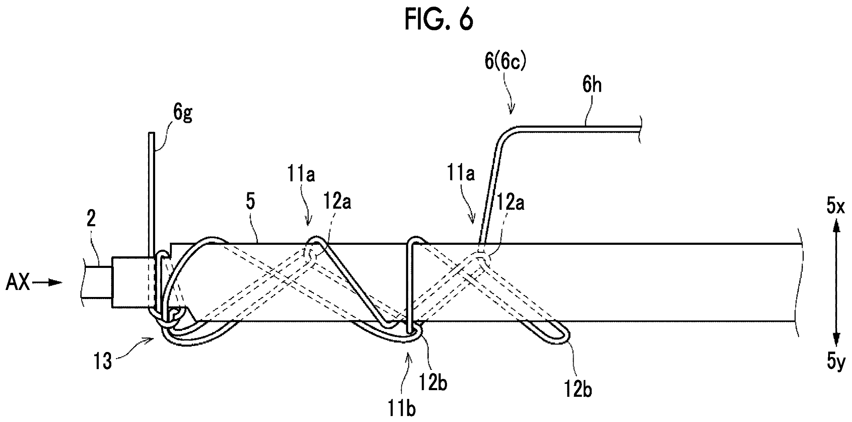

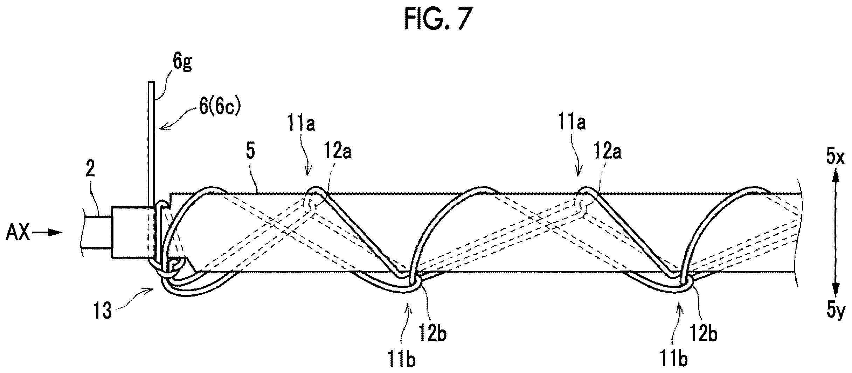

Fig. 5 is a schematic view showing a state where the first bent portion 12a is formed after the state shown in Fig. 4. Fig. 6 is a schematic view showing a state where the second bent portion 12b is formed after the state shown in Fig. 5. Fig. 7 is a schematic view showing a state where the first bent portion 12a is formed after the state shown in Fig. 6.

In Fig. 1, in order to clearly distinguish a plurality of strings 6, a proximal string 6a is shown by a dotted line, an intermediate string 6b is shown by a broken line, and a distal string 6c is shown by a solid line.

In addition, in Fig. 1, a tying shape of the string 6 is shown in a simplified rhombic lattice shape.

In addition, in Fig. 2, the stent 5 is shown such that a portion tied by the temporary fixing portions 13 provided at both end portions is selectively reduced in diameter. However, in practice, the stent 5 is reduced in diameter as a whole by the string 6. Examples of a method of reducing the stent 5 in diameter as a whole include a method of pulling the other end side 6h of the string 6 before the second temporary fixing portion 13 is formed, shortening the length of the string 6 in the tying region R, and increasing a tying load.

As shown in Figs. 1 and 2, in the manufacturing method of the stent with a string 4 according to the present embodiment, the stent 5 that is configured by a mesh body, that is passed into a tubular portion in a body of a subject, and that is expanded by its own elasticity to be able to indwell within the tubular portion and the string 6 that ties a periphery of the stent 5 to limit expansion of the contracted stent 5 and maintain the contracted state when the stent 5 is inserted into the tubular portion are prepared. In the manufacturing method, a plurality of knots are formed from one end side 6g of the string 6 to the other end side 6h of the string 6.

As shown in Figs. 3 to 5, the manufacturing method of the stent with a string 4 includes a first knot forming step and a second knot forming step. In the first knot forming step, the first bent portion 12a is formed at the string 6. In addition, the first bent portion 12a is aligned with the stent 5, and a part on the other end side 6h of the string 6 with respect to the first bent portion 12a is passed between the first bent portion 12a and the stent 5 in a counterclockwise direction (one direction) of a circumferential direction of the stent 5 to form a first knot 11a. In the second knot forming step, as shown in Fig. 4, the second bent portion 12b is formed at a part passed between the first bent portion 12a and the stent 5. In addition, as shown in Figs. 5 and 6, a part on the other end side 6h of the string 6 with respect to the second bent portion 12b in a clockwise direction (the other direction) of the circumferential direction of the stent 5, which is an opposite direction to a direction of passing through the first bent portion 12a, is passed between the second bent portion 12b and the stent 5 to form a second knot 11b.

As shown in Fig. 5, in the first knot forming step, the first knot 11a is formed on a first side 5x of the stent 5 when viewed in an axial direction AX.

As shown in Fig. 6, in the second knot forming step, the second knot 11b is formed on a second side 5y which is an opposite side to the first side 5x of the stent 5 when viewed in the axial direction AX.

As shown in Figs. 2 and 7, the manufacturing method of the stent with a string 4 is characterized in that the stent 5 is tied by the string 6 by alternately repeating the first knot forming step and the second knot forming step.

In the present embodiment, a large intestine will be described as the "tubular portion" above, but there are other digestive tubes such as a bile duct, the large intestine, and a small intestine, and blood vessels.

In addition, the fact that the second knot 11b is formed on the second side 5y which is the opposite side to the first side 5x on which the first knot 11a is formed specifically means that a center angle connecting the second knot 11b, a center axis of the stent 5, and the first knot 11a formed immediately before is not within a range of ±90 degrees (the center angle is larger than ±90 degrees but smaller than ±180 degrees) when viewed in the axial direction AX of the stent 5.

In other words, the first knot 11a and the second knot 11b adjacent to each other in the axial direction of the stent 5 are disposed at positions different from each other in a circumferential direction of the stent 5. In addition, when viewed in the axial direction AX of the stent 5, an angle formed by a line segment connecting a center of the stent 5 and the first knot 11a (a line segment extending in a radial direction of the stent 5) and a line segment connecting the center of the stent 5 and the second knot 11b (a line segment extending in the radial direction of the stent 5) exceeds 90 degrees.

In the present embodiment, each of the "first knot 11a" and the "second knot 11b" refers to a configuration where parts of the string 6 are entangled with each other by being folded back.

In other words, each of the "first knot 11a" and the "second knot 11b" is formed by a portion of the string 6 having a loop shape and another portion of the string 6 having a loop shape passing through each other's loop and hooking (entangling) each other at a portion where the extending directions of the respective portions are reversed.

In addition, the expression "between the first bent portion 12a and the stent 5" specifically means a space defined by a part where the stent 5 and the string 6 wound around the stent 5 come into contact with each other and the first bent portion 12a.

Similarly, the expression "between the second bent portion 12b and the stent 5" specifically means a space defined by the part where the stent 5 and the string 6 wound around the stent 5 come into contact with each other and the second bent portion 12b.

The manufacturing method of the stent with a string 4 is defined as alternately performing the first knot forming step and the second knot forming step.

For this reason, the manufacturing method of the stent with a string 4 is not necessarily limited to the order of the first knot forming step and the second knot forming step and may be performed in order of the second knot forming step and the first knot forming step.

With the configuration, the first knot 11a is formed on the first side 5x when viewed in the axial direction AX of the stent 5, and the second knot 11b is formed on the second side 5y when viewed in the axial direction AX of the stent 5. Accordingly, the stent 5 can be deployed (expanded) alternately on the first side 5x and the second side 5y by pulling the string 6. For this reason, a direction of a reaction force applied to the stent 5 from a narrowing portion or the like alternates between the second side 5y and the first side 5x. Therefore, compared to a case where the direction of the reaction force is constant, occurrence of a positional deviation of the stent 5 during expansion with respect to a target position for indwelling of the stent 5 can be suppressed.

In addition, as shown in Fig. 2, the stent with a string 4 according to the present embodiment includes the stent 5 that is configured by a mesh body, that is passed into the tubular portion in the body of the subject, and that is expanded by its own elasticity to be able to indwell within the tubular portion and the string 6 that ties the periphery of the stent 5 to limit expansion of the contracted stent 5 and maintain the contracted state when the stent 5 is inserted into the tubular portion.

The string 6 includes the first knot 11a formed on the first side 5x when viewed in the axial direction AX of the stent 5 and the second knot 11b formed on the second side 5y which is the opposite side to the first side 5x when viewed in the axial direction AX of the stent 5.

With the configuration, as the first knot 11a is formed on the first side 5x of the stent 5 and the second knot 11b is formed on the second side 5y of the stent 5, the stent 5 can be deployed on the first side 5x and the second side 5y as described in the manufacturing method of the stent with a string 4. Therefore, occurrence of a positional deviation of the stent 5 during the expansion caused by a reaction force applied to the stent 5 from the narrowing portion or the like with respect to the target position for indwelling of the stent 5 can be suppressed.

As shown in Fig. 1, the stent delivery device (delivery device 1) according to the present embodiment includes the stent with a string 4 and a delivery portion 10 that delivers the stent with a string 4.

With the configuration, an effect of the stent with a string 4 can be enjoyed by the delivery device 1.

### <Stent Delivery Device>

Next, the stent delivery device (delivery device 1) that delivers the stent 5 into a biological tube will be described mainly with reference to Fig. 1.

As described above, the delivery device 1 includes the stent with a string 4 and the delivery portion 10 that delivers the stent with a string 4.

The delivery portion 10 mainly includes an inner sheath 2 having a periphery to which the stent with a string 4 is attached and an outer sheath 3 that covers outer peripheries of the inner sheath 2 and the stent with a string 4 and that is configured to move relatively to the inner sheath 2 in an axis direction.

In addition, the delivery portion 10 further includes a stopper 7 that limits a movement of the stent 5 to the proximal side, a handle 8 that is gripped by the operator, and a slider 9 to which the outer sheath 3 is fixed and that is relatively slidable with respect to the handle 8 in the axis direction.

The inner sheath 2 is formed of a polyether ether ketone (PEEK) tube.

The stent 5 according to the present embodiment is a bare stent without a resin film, but may be a covered stent with a resin film. The length of the stent 5 in a natural state according to the present embodiment is, for example, 120 mm, and the outer diameter of the stent 5 in the natural state is, for example, 22 mm. A wire diameter of the stent 5 is, for example, 0.17 mm.

The length of the stent 5 may be any length such as 60, 80, and 100 mm, and the outer diameter of the stent 5 may be any size such as 18 mm in addition to 22 mm.

### <String>

The string 6 is for tying the stent 5 in a diameter-reduced state such that the stent 5 is accommodated in the outer sheath 3 when the stent 5 is transported. When the stent 5 is expanded, the string 6 is pulled by the operator to release a tying of the stent 5.

The string 6 is a string in which both or one of natural fibers or chemical fibers are twisted together. However, without being limited to such a configuration, the string 6 may be made of a metal insofar as the string 6 has a linear shape that can tie the stent 5.

As shown in Fig. 1, the plurality of strings 6 (the proximal string 6a, the intermediate string 6b, and the distal string 6c) according to the present embodiment are provided. At least some of the plurality of strings 6 tie regions of the stent 5, which are different from each other in the axial direction AX.

The proximal string 6a ties the proximal side of the stent 5, the intermediate string 6b ties an intermediate portion of the stent 5, and the distal string 6c ties the distal side of the stent 5.

As in the above configuration, as the plurality of strings 6 tie the regions of the stent 5, which are different from each other, the stent 5 can be segmentally released by releasing the tying of the plurality of strings 6 at different timings. For this reason, the stent 5 is easily and suitably indwelled at the narrowing portion.

In particular, it is suitable to provide three strings 6 since the intermediate portion can be expanded after the distal side and the proximal side of the string 6 are expanded with the narrowing portion (not shown) or the like sandwiched, but the present invention is not limited to such a configuration. For example, the string 6 may be configured by two strings including the proximal string 6a and the distal string 6c, and the string 6 may be configured by one string in a case where the segmental release is not required.

In the present embodiment, a distal end portion of the tying region R of the proximal string 6a and a proximal end portion of the tying region R of the intermediate string 6b are adjacent to each other in the axial direction of the stent 5. Similarly, a distal end portion of the tying region R of the intermediate string 6b and a proximal end portion of the tying region R of the distal string 6c are adjacent to each other in the axial direction of the stent 5.

The stent with a string 4 according to the present embodiment is indwelled in the digestive tube (for example, in the large intestine). When the plurality of strings 6 constituting the stent with a string 4 according to the present embodiment are pulled, the first knot 11a and the second knot 11b are formed, for example, such that the strings 6 are untied from an anal side to an oral side.

In other words, a manufacturer forms the first knot 11a and the second knot 11b such that the plurality of strings 6 are tied to the stent 5 from the oral side, which is the distal side of the delivery device 1 shown in Fig. 1, to the anal side, which is the proximal side being the opposite side thereto. Then, as will be described later, the manufacturer disposes the temporary fixing portion 13, which is formed at the end of a tying step of the string 6 to the stent 5, on the anal side. Accordingly, the string 6 is configured to be untied from the anal side to the oral side.

That is, the proximal string 6a, the intermediate string 6b, and the distal string 6c are configured such that, for example, ends of ties are positioned on the proximal side of the tying region R, and the stent 5 is opened from the proximal side of each tying region R.

With the configuration, by being untied from the anal side, the string 6 can be released from an anus without being folded back in the axial direction AX of the stent 5, and the string 6 can be shortened.

The expression "being untied from the anal side to the oral side" is merely an example, and a configuration where the untying is performed in reverse order is not excluded.

That is, an end of the tying of the stent 5 may be set to the distal side of the tying region R, the stent 5 may be disposed on the inner sheath 2 such that the string 6 is turned back, and the stent 5 may be opened from the distal side of the tying region (that is, untied from the oral side to the anal side).

For example, in a case of a configuration where the plurality of strings 6 provided at positions different in the axial direction AX are included, like the stent with a string 4 according to the present embodiment, a configuration where the oral side (distal side) of the stent 5 is opened from the forefront (distal side), and the anal side is opened from a base end side (proximal side) is suitable.

### [Configuration Related to Central Portion of String]

Next, a configuration related to a central portion and a configuration related to both end portions, which are related to the distal string 6c, will be mainly described with reference to Fig. 2, on behalf of the string 6. The proximal string 6a and the intermediate string 6b are configured in the same manner as the distal string 6c.

In the stent with a string 4 according to the present embodiment, as shown in Fig. 2, a plurality of first knots 11a and a plurality of second knots 11b provided at the distal string 6c are alternately formed and are each disposed along the axial direction AX of the stent 5.

Details of the first knot 11a and the second knot 11b will be described in the section of a tying method of the string 6 to be described later.

That is, the plurality of first knots 11a are provided side by side along the axial direction AX on the first side 5x (an upper side in side view shown in Fig. 2) when viewed in the axial direction AX. The plurality of second knots 11b are provided side by side along the axial direction AX on the second side 5y (a lower side in side view shown in Fig. 2) when viewed in the axial direction AX.

The expression "provided along the axial direction AX" is not limited to being provided to be completely parallel to the axial direction AX. Specifically, a center angle connecting the first knot 11a adjacent to the axial direction AX and the center axis of the stent 5 is within a range of 60 degrees when viewed in the axial direction AX of the stent 5 means being provided along the axial direction AX.

That is, in a case where the plurality of first knots 11a are provided along the axial direction AX, when viewed in the axial direction AX of the stent 5, an angle formed by a line segment connecting an axis center of the stent 5 and one first knot 11a (a line segment extending in the radial direction of the stent 5) and a line segment connecting the axis center of the stent 5 and the other first knot 11a (a line segment extending in the radial direction of the stent 5) may be 60 degrees or less. "One first knot 11a" and "the other first knot 11a" herein are the first knots 11a adjacent to each other in the axial direction AX of the stent 5 among the plurality of first knots 11a.

With the configuration, the first knot 11a and the second knot 11b can be alternately deployed along the axial direction AX of the stent 5. Therefore, deviation of the stent 5 from the target indwelling position caused by a reaction force from the narrowing portion (not shown) or the like when the stent 5 is deployed can be suppressed.

As shown in Fig. 2, a part of the string 6 that linearly extends in the axial direction AX of the stent 5 is not formed between the first knot 11a and the second knot 11b in the string 6.

That is, between the first knot 11a and the second knot 11b, the string 6 obliquely extends while having a circumferential direction component and an axial direction AX component of the stent 5 (that is, the string 6 is screwed in along an outer peripheral surface of the stent 5).

With the configuration, a part that linearly extends in the axial direction AX of the stent 5 that does not function as a member for tying the stent 5 is not formed in the string 6. Therefore, the length of the string 6 that ties the stent 5 in the circumferential direction can be shortened.

### [Configuration Related to Both End Portions of String]

As shown in Fig. 2, the temporary fixing portions 13 of the string 6 are provided at end portions (in the present embodiment, both end portions) of the tying region R where the plurality of first knots 11a and the plurality of second knots 11b are provided. The temporary fixing portions 13 mean configurations where the string 6 is not untied in a natural state but is untied when the operator pulls the string 6 instead of being fixed so that the string 6 is not untied even when the string 6 is pulled.

Details of the temporary fixing portions 13 will be described in the section of the tying method of the string 6 to be described later.

With the configuration, as the string 6 is temporarily fixed at positions different in the axial direction AX of the stent 5, the string 6 can be easily released.

It is suitable to form the temporary fixing portions 13 at both end portions of the stent 5 in the tying region R as described above, but the present invention is not limited to such a configuration. The temporary fixing portions 13 can be formed at any positions of the stent 5. For example, in a case where the tying region R of the string 6 is long in the axial direction AX, the temporary fixing portion 13 may be additionally provided at a central portion between both ends of the tying region R.

### <Tying Method>

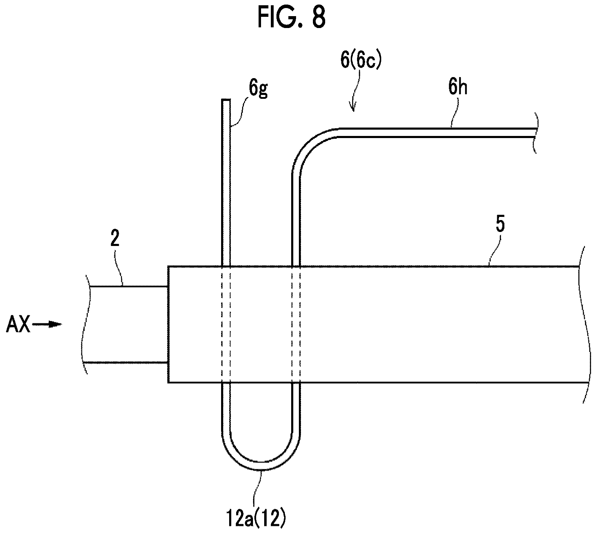

Next, the tying method of the string 6 (distal string 6c) with respect to the stent 5 will be described mainly with reference to Figs. 8 to 13 in addition to Figs. 2 to 7.

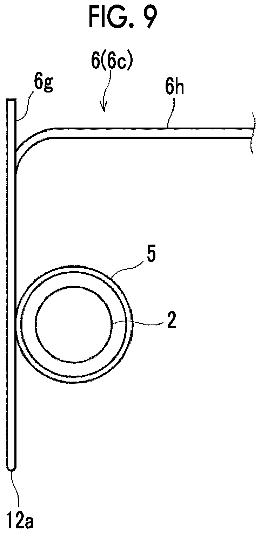

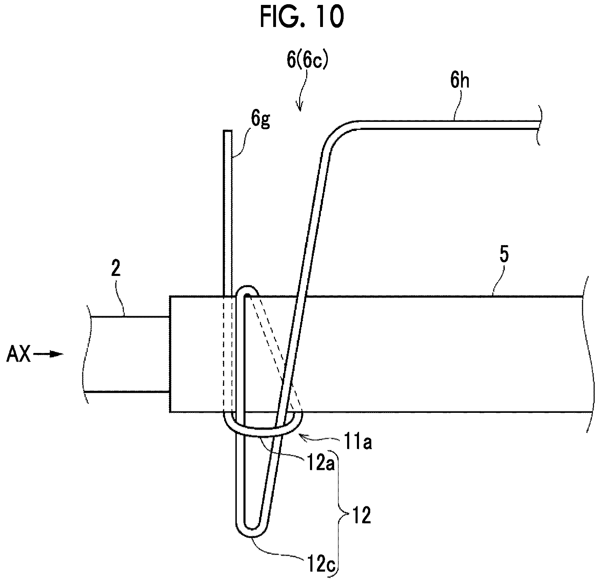

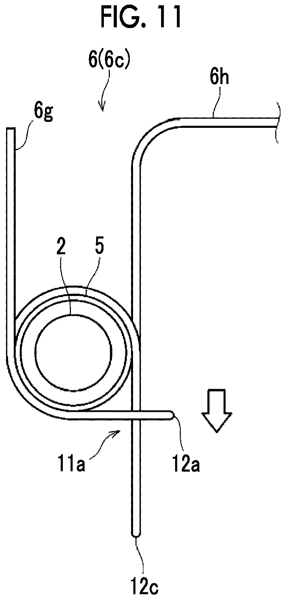

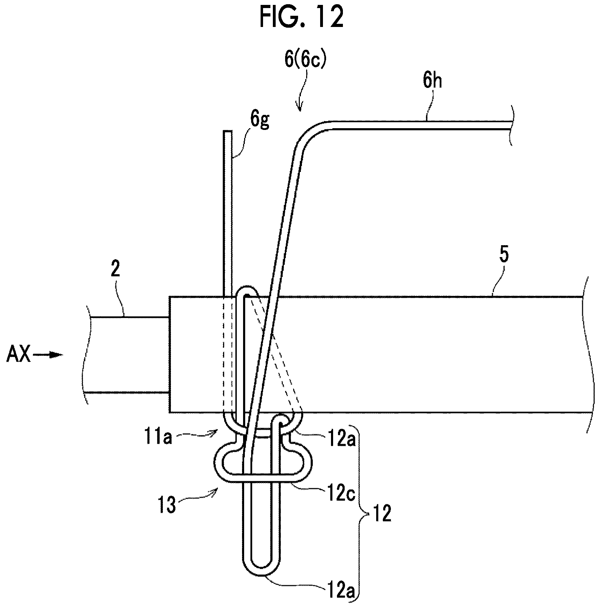

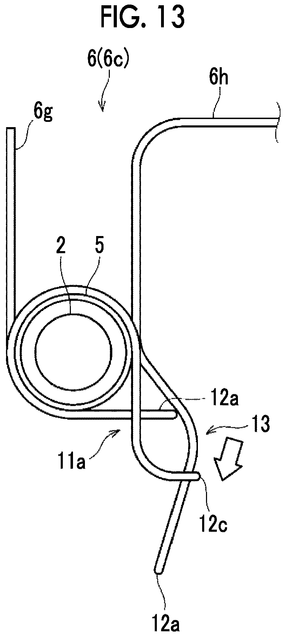

Fig. 8 is a view showing a procedure of forming the temporary fixing portion 13 and is a schematic view showing a state where the string 6 having the first bent portion 12a is aligned with the stent 5. Fig. 9 is a schematic view showing Fig. 8 viewed in the axial direction. Fig. 10 is a schematic view showing a state where a third bent portion 12c is formed after the state shown in Fig. 8. Fig. 11 is a schematic view showing Fig. 10 when viewed in the axial direction AX. Fig. 12 is a schematic view showing a state where the first bent portion 12a is formed after the state shown in Fig. 10. Fig. 13 is a schematic view showing Fig. 12 when viewed in the axial direction AX.

It is preferable that, while each of steps to be described below is performed, the one end side 6g and the other end side 6h of the string 6 are always maintained in a state of being positioned on the same side (the upper side in Figs. 2 to 15) with respect to the stent 5.

### [Tying Method on One End Side of String]

As shown in Figs. 3 and 8 to 13, the manufacturing method of the stent with a string 4 includes a temporarily fixing step of temporarily fixing the string 6 to the stent 5.

First, as shown in Figs. 8 and 9, in the temporarily fixing step, the string 6 is aligned with a side peripheral surface of the stent 5, and the string 6 is disposed such that a bent portion 12 (first bent portion 12a) of the string 6 protrudes outward from the stent 5 in side view shown in Fig. 8.

In the tying method on the one end side 6g of the string 6, one circumferential direction around the stent 5 is defined as the clockwise direction, and the other circumferential direction around the stent 5 is defined as the counterclockwise direction (to be described later), but this is merely an example and the present invention is not limited thereto.

The first knot 11a is formed by passing the part on the other end side 6h of the string 6 with respect to the first bent portion 12a between the first bent portion 12a and the stent 5 in the clockwise direction (for example, the clockwise direction indicated by a block arrow of Fig. 11) of the circumferential direction of the stent 5 as shown in Figs. 10 and 11.

After the first knot 11a is formed, as shown in Fig. 10, the third bent portion 12c is formed at the part of the string 6, which is passed between the first bent portion 12a and the stent 5.

The temporary fixing portion 13 is formed by passing a part on the other end side 6h of the string 6 with respect to the third bent portion 12c between the third bent portion 12c and the first knot 11a in the same direction as the direction of passing through the first bent portion 12a, in the clockwise direction of the circumferential direction of the stent 5 (for example, the clockwise direction shown in Fig. 13) as shown in Figs. 12 and 13. Further, the first bent portion 12a is formed again at a part passed between the third bent portion 12c and the stent 5.

More specifically, when forming the temporary fixing portion 13, a part which is on the other end side 6h of the string 6 with respect to the third bent portion 12c and which is on the other end side 6h with respect to the first knot 11a is passed between the third bent portion 12c and the first knot 11a.

In this manner, the temporary fixing portion 13 shown in Fig. 3 is formed.

Through the steps described above, for example, a portion between one end and the other end of the string 6 is wound around the stent 5 in the other circumferential direction around the stent 5 (for example, wound around the stent 5 by approximately half a periphery), and a tip side portion (a lower end portion in Fig. 8) of the wound portion is the bent portion 12 (the first bent portion 12a: hereinafter, referred to as a first loop portion in some cases). Further, for example, a part on the other end side 6h of the string 6 with respect to the first loop portion is wound around the stent 5 (for example, wound around the stent 5 to make approximately one turn) in one circumferential direction around the stent 5 and is passed between the first loop portion and the stent 5. The passed portion is a new bent portion 12 (the third bent portion 12c shown in Fig. 10: hereinafter, referred to as a second loop portion in some cases). Further, for example, a part on the other end side 6h of the string 6 with respect to the second loop portion is passed through the second loop portion, and the passed portion is a new bent portion 12 (the lower first bent portion 12a shown in Figs. 12 and 13: hereinafter, referred to as a third loop portion in some cases). By tying the string 6 wound around the stent 5 in this manner to the stent 5 so that the third loop portion remains, the temporary fixing portion 13 is formed.

With the configuration, by forming the temporary fixing portion 13, workability of tying the string 6 can be improved.

### [Tying Method of Central Portion of String]

Next, as shown in Fig. 4, the first bent portion 12a (third loop portion) that is provided at the temporary fixing portion 13 and that protrudes from the first knot 11a is aligned with the axial direction of the stent 5. Then, the first knot 11a is formed by passing the part on the other end side 6h of the string 6 with respect to the first bent portion 12a between the first bent portion 12a and the stent 5 in the counterclockwise direction in the circumferential direction of the stent 5 (first knot forming step) .

In the tying method of the central portion of the string 6, the one circumferential direction around the stent 5 is defined as the counterclockwise direction, and the other circumferential direction around the stent 5 is defined as the clockwise direction. However, this is merely an example, and the present invention is not limited thereto.

As shown in Fig. 4, the second bent portion 12b is formed at the part passed between the first bent portion 12a and the stent 5.

As shown in Figs. 5 and 6, the second knot 11b is formed by passing the part on the other end side 6h of the string 6 with respect to the second bent portion 12b between the second bent portion 12b and the stent 5 in the clockwise direction of the circumferential direction of the stent 5, which is the opposite direction to the direction of passing through the first bent portion 12a (second knot forming step) .

Through the steps described above, for example, a part on the other end side 6h of the string 6 with respect to the third loop portion is wound around the stent 5 while being screwed in to make less than one turn in the other circumferential direction around the stent 5 and is passed between the third loop portion and the stent 5, and the passed portion is a new bent portion 12 (the second bent portion 12b: hereinafter, referred to as a fourth loop portion in some cases) (Fig. 4).

Further, for example, a part on the other end side 6h of the string 6 with respect to the fourth loop portion is wound around the stent 5 while being screwed in to make less than one turn in the one circumferential direction around the stent 5 and is passed between the fourth loop portion and the stent 5. The passed portion is a new bent portion 12 (the lower first bent portion 12a shown in Fig. 5: hereinafter, referred to as a fifth loop portion), and in this case, the first knot 11a shown in Fig. 5 is also formed.

Further, for example, a part on the other end side 6h of the string 6 with respect to the fifth loop portion is wound around the stent 5 while being screwed in to make less than one turn in the other circumferential direction around the stent 5 and is passed between the fifth loop portion and the stent 5. The passed portion is a new bent portion 12 (the right second bent portion 12b shown in Fig. 6: hereinafter, referred to as a sixth loop portion), and in this case, the second knot 11b shown in Fig. 6 is also formed.

Then, as shown in Figs. 6 and 7, the stent 5 is tied by the central portion of the string 6 by repeating first knot forming step and the second knot forming step toward the proximal side (the other end side) of the stent 5.

The number of times of the first knot forming step (the number of first knots 11a at the central portion of the string 6) and the number of times of the second knot forming step (the number of second knots 11b at the central portion of the string 6) may be the same or one may be larger than the other by one time (one knot).

### [Temporary Fixing on Other End Side]

A temporary fixing portion 13 (the temporary fixing portion 13 on the other end side is not shown) similar to the temporary fixing portion 13 on the one end side is formed also on the other end side of the string 6.

The other end side 6h drawn out from the temporary fixing portion 13 extends to the handle 8. For this reason, by pulling the other end side 6h of the string 6 with the handle 8, the operator can untie the temporary fixing portion 13, the first knot 11a, and the second knot 11b and can expand the stent 5. More specifically, by pulling the other end side 6h of the string 6, first, the temporary fixing portion 13 on the other end side 6h of the two temporary fixing portions 13 is untied, next, the central portion of the string 6 is untied, and finally, the temporary fixing portion 13 on the one end side 6g is released.

In particular, in the present embodiment, the handle 8 is configured to separately pull each of the proximal string 6a, the intermediate string 6b, and the distal string 6c.

### <Modification Example>

The tying method of the string 6 according to the embodiment is an example, and various types of changes can be made.

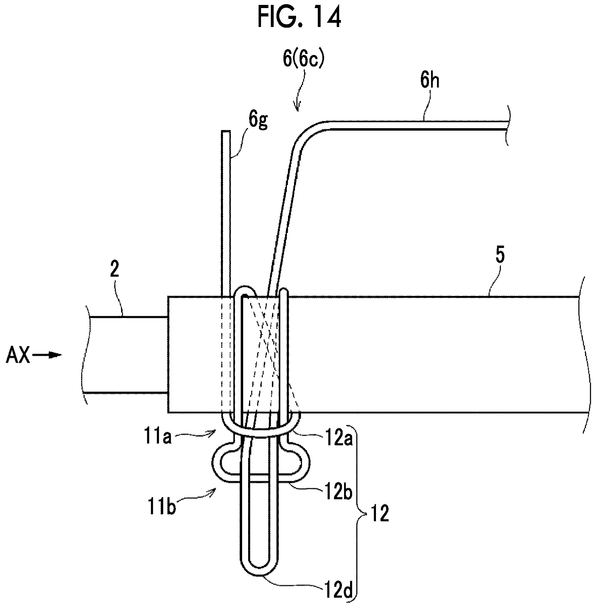

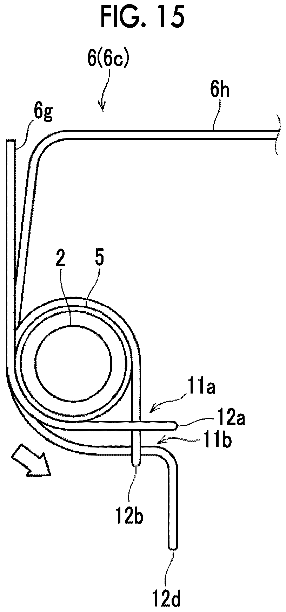

For example, a temporary fixing portion according to a modification example will be mainly described with reference to Figs. 14 and 15.

Fig. 14 is a view showing a procedure of forming the temporary fixing portion according to the modification example and is a schematic view showing a state where a fourth bent portion 12d is formed after the state shown in Fig. 8. Fig. 15 is a schematic view showing Fig. 14 when viewed in the axial direction AX.

In a step of forming the temporary fixing portion according to the modification example, as in the procedure shown in Figs. 10 and 11, after the state shown in Fig. 8, the part on the other end side 6h of the string 6 with respect to the first bent portion 12a is passed through a space between the first bent portion 12a and the stent 5 in the clockwise direction when viewed in the axial direction AX shown in Fig. 15, and the first knot 11a is formed. The second bent portion 12b (the same as the third bent portion 12c shown in Fig. 10) is formed while forming the first knot 11a. Further, the part on the other end side 6h of the string 6 with respect to the second bent portion 12b is passed through a space between the second bent portion 12b and the stent 5 in the counterclockwise direction when viewed in the axial direction AX shown in Fig. 15, and the second knot 11b is formed.

As shown in Figs. 14 and 15, after the second knot 11b is formed, the fourth bent portion 12d is formed at the part passed between the second bent portion 12b and the stent 5. Then, the temporary fixing portion (the same as the temporary fixing portion 13 shown in Figs. 12 and 13 other than a passing direction of the part on the other end side 6h of the string 6) is formed by passing a part on the other end side 6h of the string 6 with respect to the fourth bent portion 12d between the fourth bent portion 12d and the second knot 11b in the same direction as a direction of passing through the second bent portion 12b, in the clockwise direction of the circumferential direction of the stent 5 (for example, the counterclockwise direction shown in Fig. 15). Further, the second bent portion 12b is formed again at a part passed between the fourth bent portion 12d and the stent 5.

More specifically, when forming the temporary fixing portion, a part which is on the other end side 6h of the string 6 with respect to the fourth bent portion 12d and which is on the other end side 6h with respect to the second knot 11b is passed between the fourth bent portion 12d and the first knot 11a.

As shown in Figs. 14 and 15, the temporarily fixing step is performed after one or more first knots 11a and one or more second knots 11b are alternately formed at the same positions in the axial direction AX of the stent 5.

The expression "the same positions in the axial direction AX", which are positions where the one or more first knots 11a and the one or more second knots 11b are alternately formed, means the same positions in a certain region where a force of tying the temporary fixing portion is applied and means the same positions at a certain length.

With the configuration, tying strength can be locally improved by performing temporarily fixing after the one or more first knots 11a and the one or more second knots 11b are alternately formed at the same positions.

As described above, the temporary fixing portion 13 and the temporary fixing portion according to the modification example are formed by continuously passing the part on the other end side 6h of the string 6 in the same direction as the circumferential direction of the stent 5 with respect to the bent portion 12 and the first knot 11a or the second knot 11b. For this reason, before the part on the other end side 6h of the string 6 is continuously passed continuously therefrom, the number of times of forming the first knot 11a and the second knot 11b can be set in any manner. Although work hours are required by forming a large number of first knots 11a and second knots 11b, the force of tying the temporary fixing portion is stronger.

In addition, when the string 6 is fixed by the temporary fixing portion 13 and the temporary fixing portion according to the modification example, work requires less effort than in a case where other members are used, which is suitable. However, the temporary fixing portion 13 and the temporary fixing portion according to the modification example are not limited to being formed by this means, and the formation may be performed with an adhesive or the like.

The embodiment includes the following technical ideas.
(1) A manufacturing method of a stent with a string in which a stent that is passed in a tubular portion in a body of a subject and that is expanded by elasticity of the stent to be able to indwell within the tubular portion and a string that ties a periphery of the stent such that expansion of the contracted stent is limited to maintain a contracted state when inserting the stent into the tubular portion are prepared, and a plurality of knots from one end side of the string to the other end side of the string are formed, the manufacturing method including:
   a first knot forming step of forming a first bent portion at the string and forming a first knot by aligning the first bent portion with the stent and by passing a part on the other end side of the string with respect to the first bent portion between the first bent portion and the stent in one circumferential direction around the stent; and
   a second knot forming step of forming a second bent portion at the part passed between the first bent portion and the stent and forming a second knot by passing a part on the other end side of the string with respect to the second bent portion between the second bent portion and the stent in the other circumferential direction around the stent, which is an opposite direction to a direction of passing the first bent portion,
   in which in the first knot forming step, the first knot is formed on a first side when viewed in an axial direction of the stent,
   in the second knot forming step, the second knot is formed on a second side, which is an opposite side to the first side, when viewed in the axial direction of the stent, and
   the stent is tied with the string by alternately repeating the first knot forming step and the second knot forming step.
(2) The manufacturing method of a stent with a string according to (1), further including:
   a temporarily fixing step of temporarily fixing the string to the stent,
   in which in the temporarily fixing step, after the first knot is formed, a third bent portion is formed at the part of the string, which is passed between the first bent portion and the stent, and a temporary fixing portion is formed by passing a part on the other end side of the string with respect to the third bent portion between the third bent portion and the first knot in the one circumferential direction around the stent, which is the same direction as the direction of passing the first bent portion, and the first bent portion is formed again at a part passed between the third bent portion and the stent.
(3) The manufacturing method of a stent with a string according to (1), further including:
   a temporarily fixing step of temporarily fixing the string to the stent,
   in which in the temporarily fixing step, after the second knot is formed, a fourth bent portion is formed at the part of the string, which is passed between the second bent portion and the stent, and a temporary fixing portion is formed by passing a part on the other end side of the string with respect to the fourth bent portion between the fourth bent portion and the second knot in the other circumferential direction around the stent, which is the same direction as a direction of passing the second bent portion, and the second bent portion is formed again at a part passed between the fourth bent portion and the stent.
(4) The manufacturing method of a stent with a string according to (2) or (3),
   in which the temporarily fixing step is performed after one or more first knots and one or more second knots are alternately formed at the same positions of the stent in the axial direction.
(5) A stent with a string including:
   a stent that is passed in a tubular portion in a body of a subject and that is expanded by elasticity of the stent to be able to indwell within the tubular portion; and
   a string that ties a periphery of the stent such that expansion of the contracted stent is limited to maintain a contracted state when inserting the stent into the tubular portion,
   in which the string includes a first knot formed on a first side when viewed in an axial direction of the stent, and a second knot formed on a second side, which is an opposite side to the first side, when viewed in the axial direction of the stent.
(6) The stent with a string according to (5),
   in which a plurality of the first knots and a plurality of the second knots are alternately formed and are each disposed along the axial direction of the stent.
(7) The stent with a string according to (5) or (6),
   in which a part linearly extending in the axial direction of the stent is not formed between the first knot and the second knot of the string.
(8) The stent with a string according to (7),
   in which the stent with a string is a stent with a string manufactured under the manufacturing method of a stent with a string according to (4), and
   the temporary fixing portion is provided at an end portion of a region where the plurality of first knots and the plurality of second knots are provided.
(9) A stent delivery device including:
   the stent with a string according to any one of (5) to (8); and
   a delivery portion that delivers the stent with a string.
(10) The stent delivery device according to (9),
   in which a plurality of the strings are provided, and
   at least some of the plurality of strings tie regions different in an axial direction of the stent.
(11) The stent delivery device according to (9) or (10),
   in which the stent with a string is indwelled in a digestive tube, and
   when the plurality of strings are pulled, the first knot and the second knot are formed such that the strings are untied from an anal side to an oral side.

### Reference Signs List

1: delivery device (stent delivery device)
2: inner sheath
3: outer sheath
4: stent with string
5: stent
5x: first side
5y: second side
6: string
6a: proximal string
6b: intermediate string
6c: distal string
6g: one end side
6h: other end side
7: stopper
8: handle
9: slider
10: delivery portion
11a: first knot
11b: second knot
12: bent portion
12a: first bent portion
12b: second bent portion
12c: third bent portion
12d: fourth bent portion
13: temporary fixing portion
AX: axial direction
R: region

## Claims

1. A manufacturing method of a stent with a string in which a stent that is passed in a tubular portion in a body of a subject and that is expanded by elasticity of the stent to be able to indwell within the tubular portion and a string that ties a periphery of the stent such that expansion of the contracted stent is limited to maintain a contracted state when inserting the stent into the tubular portion are prepared, and a plurality of knots from one end side of the string to the other end side of the string are formed, the manufacturing method comprising:
a first knot forming step of forming a first bent portion at the string and forming a first knot by aligning the first bent portion with the stent and by passing a part on the other end side of the string with respect to the first bent portion between the first bent portion and the stent in one circumferential direction around the stent; and
a second knot forming step of forming a second bent portion at the part passed between the first bent portion and the stent and forming a second knot by passing a part on the other end side of the string with respect to the second bent portion between the second bent portion and the stent in the other circumferential direction around the stent, which is an opposite direction to a direction of passing the first bent portion,
wherein in the first knot forming step, the first knot is formed on a first side when viewed in an axial direction of the stent,
in the second knot forming step, the second knot is formed on a second side, which is an opposite side to the first side, when viewed in the axial direction of the stent, and
the stent is tied with the string by alternately repeating the first knot forming step and the second knot forming step.

2. The manufacturing method of a stent with a string according to Claim 1, further comprising:
a temporarily fixing step of temporarily fixing the string to the stent,
wherein in the temporarily fixing step, after the first knot is formed, a third bent portion is formed at the part of the string, which is passed between the first bent portion and the stent, and a temporary fixing portion is formed by passing a part on the other end side of the string with respect to the third bent portion between the third bent portion and the first knot in the one circumferential direction around the stent, which is the same direction as the direction of passing the first bent portion, and the first bent portion is formed again at a part passed between the third bent portion and the stent.

3. The manufacturing method of a stent with a string according to Claim 1, further comprising:
a temporarily fixing step of temporarily fixing the string to the stent,
wherein in the temporarily fixing step, after the second knot is formed, a fourth bent portion is formed at the part of the string, which is passed between the second bent portion and the stent, and a temporary fixing portion is formed by passing a part on the other end side of the string with respect to the fourth bent portion between the fourth bent portion and the second knot in the other circumferential direction around the stent, which is the same direction as a direction of passing the second bent portion, and the second bent portion is formed again at a part passed between the fourth bent portion and the stent.

4. The manufacturing method of a stent with a string according to Claim 2 or 3,
wherein the temporarily fixing step is performed after one or more first knots and one or more second knots are alternately formed at the same positions of the stent in the axial direction.

5. A stent with a string comprising:
a stent that is passed in a tubular portion in a body of a subject and that is expanded by elasticity of the stent to be able to indwell within the tubular portion; and
a string that ties a periphery of the stent such that expansion of the contracted stent is limited to maintain a contracted state when inserting the stent into the tubular portion,
wherein the string includes a first knot formed on a first side when viewed in an axial direction of the stent, and a second knot formed on a second side, which is an opposite side to the first side, when viewed in the axial direction of the stent.

6. The stent with a string according to Claim 5,
wherein a plurality of the first knots and a plurality of the second knots are alternately formed and are each disposed along the axial direction of the stent.

7. The stent with a string according to Claim 6,
wherein a part linearly extending in the axial direction of the stent is not formed between the first knot and the second knot of the string.

8. The stent with a string according to Claim 7,
wherein the stent with a string is a stent with a string manufactured under the manufacturing method of a stent with a string according to Claim 4, and
the temporary fixing portion is provided at an end portion of a region where the plurality of first knots and the plurality of second knots are provided.

9. A stent delivery device comprising:
the stent with a string according to any one of Claims 5 to 7; and
a delivery portion that delivers the stent with a string.

10. The stent delivery device according to Claim 9,
wherein a plurality of the strings are provided, and
at least some of the plurality of strings tie regions different in an axial direction of the stent.

11. The stent delivery device according to Claim 9,
wherein the stent with a string is indwelled in a digestive tube, and
when the plurality of strings are pulled, the first knot and the second knot are formed such that the strings are untied from an anal side to an oral side.
